Europäisches Patentamt

European Patent Office

Office européen des brevets

(11) Veröffentlichungsnummer: **0 140 203**
**A2**

## (12) EUROPÄISCHE PATENTANMELDUNG

(21) Anmeldenummer: **84111863.1**

(22) Anmeldetag: **04.10.84**

(51) Int. Cl.⁴: **A 61 K 31/715**
**A 61 K 31/60, C 08 B 37/16**

(30) Priorität: **18.10.83 DE 3337802**

(43) Veröffentlichungstag der Anmeldung:
**08.05.85 Patentblatt 85/19**

(84) Benannte Vertragsstaaten:
**AT DE**

(71) Anmelder: **Merck Patent Gesellschaft mit beschränkter Haftung**
**Frankfurter Strasse 250**
**D-6100 Darmstadt(DE)**

(72) Erfinder: **Basedow, Arno, Dr.**
**Elisabethenstrasse 25**
**D-6368 Bad Vilbel(DE)**

(54) Pharmazeutische Zubereitung.

(57) Die Erfindung betrifft eine pharmazeutische Zubereitung in fester Form enthaltend Acetylsalicylsäure, gekennzeichnet durch einen zusätzlichen Gehalt an β-Cyclodextrin.

EP 0 140 203 A2

Croydon Printing Company Ltd.

— 1 —

Pharmazeutische Zubereitung

Die Erfindung betrifft pharmazeutische Zubereitungen in fester Form, die Acetylsalicylsäure (ASS) enthalten. Insbesondere betrifft die Erfindung pharmazeutische Zubereitungen in fester Form, die neben ASS noch Codeinphosphat enthalten.

Derartige Zubereitungen sind vielfach bekannt. Der Wirkstoff ASS ist jedoch relativ instabil, da daraus durch Hydrolyse Essigsäure abgespalten wird.

In Kombinationspräparaten, die ASS und Codeinphosphat enthalten, bewirkt die gebildete Essigsäure eine e - Zersetzung des Codeinphosphats unter Bildung von Acetylcodein und verschiedenen Nebenprodukten; das verursacht eine starke Verfärbung der Zubereitung. Kombinationspräparate, die ASS und Codeinphosphat enthalten, sind daher schlecht haltbar, insbesondere, wenn die Wirkstoffe hoch dosiert sind.

Der Erfindung lag die Aufgabe zugrunde, die Haltbarkeit fester ASS-haltiger pharmazeutischer Zubereitungen, insbesondere solcher, die zusätzlich Codeinphosphat enthalten, zu verbessern.

Zur Lösung dieser Aufgabe wurden zahlreiche Versuche unternommen, die keinen oder nur geringen Erfolg brachten. So verbesserte ein Zusatz von Säuren wie Weinsäure, Citronensäure, Äpfelsäure, Adipinsäure, Fumarsäure oder Ascorbinsäure die Haltbarkeit nicht, ebensowenig ein Zusatz von basischen Komponenten oder
Antacida wie Calcium- oder Natriumcarbonat, Magnesium-
oder Aluminiumoxid, Magaldrat, Aluminiumhydroxid-Magnesiumcarbonat-Gel, Aluminiumhydroxid-Gel, Tricalciumphosphat, Aluminiumdihydroxyglycinat oder von wasserbindenden Substanzen wie Sucralfat (getrocknet), Gluconolacton oder wasserfreiem Natriumsulfat. Weder die
Verwendung von pulverförmigen Hartfetten noch die von
Silikonöl, auf Talk aufgezogen, gaben befriedigende
Ergebnisse.

Es wurde gefunden, daß dagegen überraschenderweise ein
Zusatz von ß-Cyclodextrin, zweckmäßig in Mengen von
etwa 0,2 bis etwa 50, vorzugsweise etwa 5 bis 10% vom
Gewicht der festen Zubereitung, feste ASS enthaltende
pharmazeutische Zubereitungen stabilisiert und haltbar
macht. Dadurch ist die oben angegebene Aufgabe gelöst.

Es ist bekannt, daß ß-Cyclodextrin in Lösung mit vielen
Substanzen Einschlußverbindungen bildet, und daß die
Hydrolyse von ASS in wässeriger Lösung in Gegenwart
von ß-Cyclodextrin verlangsamt wird (vgl. Chemical
Abstracts 72. (1970) 59028.e). Eine Stabilisierung
von festen ASS-haltigen Zubereitungen durch Zusatz
relativ geringer Mengen von ß-Cyclodextrin ist dagegen
neu und war im Hinblick auf die bekannten Eigenschaften des ß-Cyclodextrins auch nicht zu erwarten.

Gegenstand der Erfindung ist dementsprechend eine
pharmazeutische Zubereitung in fester Form, enthaltend
ASS, gekennzeichnet durch einen zusätzlichen Gehalt
an ß-Cyclodextrin. Ferner ist Gegenstand der Erfindung
ein Verfahren zur Stabilisierung von ASS enthaltenden
pharmazeutischen Zubereitungen in fester Form, dadurch
gekennzeichnet, daß man den Zubereitungen ß-Cyclodextrin zusetzt.

- 3 -

Als Zubereitungen kommen in erster Linie Tabletten, Filmtabletten, Dragees und Kapseln, insbesondere Hartgelatinekapseln in Betracht. Die Zubereitungen können übliche Träger- und/oder Hilfsstoffe enthalten, beispielsweise Füllmittel, Spreng- und Gleitmittel, im einzelnen z.B. Kohlehydrate wie Lactose oder Stärke, Stearinsäure und ihre Salze wie Calcium- oder Magnesiumstearat, Talk, Cellulose oder Cellulosederivate.

Die Zubereitungen können auch weitere Wirkstoffe enthalten, beispielsweise solche, mit denen ASS bekanntermaßen kombiniert wird, vorzugsweise Codeinphosphat, weiterhin z.B. andere Analgetika wie Paracetamol, Phenacetin, Propyphenazon, Butalbital, Salicylamid, Ethenzamid; Antacida wie Aluminiumglycinat oder basisches Magnesiumcarbonat; ferner z.B. Coffein, Phenobarbital, Pentobarbital, Cyclobarbital, Benzylmandelat, Vitamine wie Ascorbinsäure oder Thiamin, Meprobamat, Chinin.

ASS kann in den Zubereitungen auch in Form von Salzen vorliegen, z.B. als Aluminium- oder Magnesiumacetylsalicylat oder Lysin-mono-acetylsalicylat.

Ein besonderer Vorteil des Zusatzes von ß-Cyclodextrin liegt darin, daß der Wassergehalt der festen Zubereitung relativ hoch liegen kann (bis ca. 2,5%) im Gegensatz zur ß-cyclodextrinfreien Form, bei der ein Wassergehalt von 0,4 - 0,5% schon eine Zersetzung bewirkt. Auch die Temperaturstabilität wird durch Zusatz von ß-Cyclodextrin deutlich verbessert.

ß-Cyclodextrin kann wie jeder Hilfsstoff in die
Tablettenrezeptur eingearbeitet werden. Es kann
als Pulver oder feinkristallines Material einem
direkt verpreßbaren Gemisch zugegeben werden, es
kann aber auch durch Feuchtgranulation in Granulate
eingearbeitet werden. ß-Cyclodextrin beeinflußt die
Zerfallszeit der fester Darreichungsformen nicht;
es beeinträchtigt auch nicht die in-vivo-Freisetzung
der Wirkstoffe.

Merck Patent Gesellschaft
mit beschränkter Haftung
    D a r m s t a d t


Patentansprüche:


1. Pharmazeutische Zubereitung in fester Form
   enthaltend Acetylsalicylsäure, gekennzeichnet
   durch einen zusätzlichen Gehalt an ß-Cyclodextrin.


2. Verfahren zur Stabilisierung von Acetylsalicylsäure enthaltenden pharmazeutischen Zubereitungen
   in fester Form, dadurch gekennzeichnet, daß man
   den Zubereitungen ß-Cyclodextrin zusetzt.

Beispiel 1:        Tablette

Eine Tablette mit 13 mm Durchmesser, ca. 4 mm Höhe und
einer Bruchfestigkeit von ca. 60 N enthält:

| | |
|---|---|
| ASS | 500 mg |
| Codeinphosphat-hemihydrat | 30 mg |
| Mikrokristalline Cellulose | 30 mg |
| Maisstärke | 30 mg |
| ß-Cyclodextrin | 30 mg |
| Talkum | 30 mg. |

Beispiel 2:        Tablette:

Eine Tablette mit 13 mm Durchmesser, ca. 4 mm Höhe und
einer Bruchfestigkeit von ca. 50 N enthält:

| | |
|---|---|
| ASS | 250 mg |
| Paracetamol | 200 mg |
| Coffein | 50 mg |
| Mikrokristalline Cellulose | 70 mg |
| Maisstärke | 30 mg |
| ß-Cyclodextrin | 20 mg |
| Talkum | 30 mg. |

Beispiel 3:        Tablette

Eine Tablette mit 12 mm Durchmesser, ca. 4 mm Höhe
und einer Bruchfestigkeit von ca. 50 N enthält:

| | |
|---|---|
| ASS | 400 mg |
| Mikrokristalline Cellulose | 60 mg |
| Maisstärke | 45 mg |
| ß-Cyclodextrin | 10 mg |
| Stearinsäure, gepulvert | 5 mg. |